# EUROPEAN PATENT SPECIFICATION

(11) **EP 0 335 136 B1**
(45) Date of publication and mention of the grant of the patent: **08.06.1994**
(21) Application number: 89103861.4
(22) Date of filing: 06.03.1989
(51) Int. Cl.: G01N 33/84

(54) **Calcium assay reagent**
Kalziumtestreagenz
Réactif d'essai de calcium

(30) Priority: 28.03.1988 US 173978
(43) Date of publication of application: 04.10.1989
(73) Proprietor: ABBOTT LABORATORIES, Abbott Park, Illinois 60064-3500 (US)
(72) Inventor: Hissami, Obaid, Bedford, TX 76021 (US)
(74) Representative: Modiano, Guido, Dr.-Ing.

(56) References cited:
- EP-A- 0 067 041
- EP-A- 0 097 472
- EP-A- 0 202 093
- DE-A- 3 329 952
- US-A- 3 822 116
- US-A- 3 938 954

## Description

### BACKGROUND OF THE INVENTION

The quantitative determination of calcium in serum samples is widely carried out by the modified method of Connerty and Briggs, in which a dye, o-cresolphthalein complexone (CPC), reacts with calcium in an alkaline medium. This reaction gives rise to a dye-calcium complex whose absorbance is directly proportional to the concentration of calcium in the sample. Typically, the absorbance is monitored at wavelengths around 570 nm.

In general, calcium reagents based on CPC methodology consist of two distinct components. The first component, usually referred to as the "dye", contains various concentrations of CPC. The second component is called the "buffer" and is composed of number of divergent buffer systems. A "working reagent" is prepared by combining in a number of combinations, a dye and buffer component.

Previously, the CPC calcium procedure was run with a diethylamine buffer system. More recently, Moorehead and Biggs reported that an improved procedure was obtained by replacing the diethylamine buffer with 2-amino-2-mehtyl-1-propanol and changing the pH of the rection environment from 12 to 10. Moorehead, W. R., Biggs, H. G., "2-Amino-2-Methyl-1-Propanol as the Alkalizing Agent in an Improved Continuous-Flow Cresolphthalein Complexon Procedure for Calcium in Serum", Clin. Chem. 20/11, 1458-60 (1974). The authors indicated that these modifications resulted in increased sensitivity, improved baseline stability, freedom from magnesium interference, and no blanking problems. While this substitution of buffer may have improved the CPC reagent, reagent stability, per se, remained a problem.

Reagent stability becomes an even more important factor when one considers that the spectrophotometric determination of calcium in serum by the CPC methods is a common procedure for the measurement of this analyte, so common that it has been adapted to many automated analyzers. In automated systems, reagent stability can translate into such important characteristics as "working reagent stability" and "calibration frequency". Working reagent stability is defined as the time interval between the time the reagent is prepared and the time reagent performance is unacceptable. This unacceptability may still be present even though the reagent has been freshly calibrated. In other terms, the reagent chemistry is unable to function properly. Calibration frequency refers to the time interval during which the reagent will perform acceptably without the need to recalibrate. Calibration frequency is, de facto, a measure of reagent stability, in that a highly stable reagent will not require recalibration as often as a metastable or unstable reagent. To circumvent this problem, a number of diagnostic reagent manufacturers have either resorted to a unit dose type of calcium assay or to an "aging" of the calcium working reagent. Aging involves preparation of the working reagent, allowing the working reagent to incubate for a predetermined time interval prior to utilization.

Relevant to the background of the present invention is EP-A-0 067 041 which discloses the use of a carbonate and/or bicarbonate buffer system in a method for measuring the amount of calcium in a sample using an o-cresolphthalein complexone dye reagent. EP-A-0 067 041 also discloses the use of 8-oxyquinoline or 8-oxyquinoline-5-sulfonic acid as a masking agent for magnesium.

DE-A-3 329 952 discloses the use of sodium desoxycholate for reducing lipid turbidity in a sample.

EP-A-0 202 093 discloses the use of a reagent comprising a buffering agent, a nonionic surfactant, cyclodextrin, lipase and water for reducing turbidity in biological fluids.

EP-A-0 097 472 discloses the use of a nonionic surfactant for reducing turbidity in a method for determining calcium in a biological fluid, such method involving o-cresolphthalein complexone and a masking agent for magnesium, in particular 8-hydroxyquinoline.

### SUMMARY OF THE INVENTION

The present invention is directed to a stable calcium reagent for the spectrophotometric determination of calcium in a test sample, said stable calcium reagent characterized by:
(a) a dye component including:
   (i) o-cresolphthalein complexone; and
   (ii) a magnesium-interference reducing amount of 8-quinolinol sulfate; in admixture with
(b) a buffer component consisting essentially of:
   (i) a reagent-stability improving amount of a carbonate or bicarbonate buffer selected from the group consisting of sodium carbonate, sodium bicarbonate, potassium carbonate, potassium bicarbonate, lithium carbonate and lithium bicarbonate; and
   (ii) a lipid-turbidity reducing amount of nonylphenoxypoly(ethyleneoxy) ethanol in combination with a salt of a cholic acid, said buffer component having a pH of from 9.0 to 10.5.

This particular combination of a bicarbonate or carbonate buffer with the traditional dye yields a working calcium reagent system having both excellent working reagent stability and extended calibration frequency.

Generally, the carbonate or bicarbonate buffer is combined with the dye reagent to yield a working reagent with a pH of 9.0 to 10.5. Preferred carbonate or bicarbonate buffers comprise sodium carbonate or sodium bicarbonate. Preferably, the buffer is mixed with the dye in approximately equal volumes.

Moreover, the calcium reagent of the present invention is optimized for additional performance characteristics. For example, the system can include Igepal® CO-530 as the nonionic surfactant and sodium cholate as the salt of a cholic acid to minimize lipemic interference. 8-quinolol sulfate has been selected to reduce magnesium interference.

### DETAILED DESCRIPTION OF THE INVENTION

The metal-complexing dye o-cresolphthalein complexone (CPC) binds to calcium in alkaline solution to form a chromophore, whose absorbance is measured spectrophotometrically at about 570 nm. This absorbance can also be determined bichromatically at about 570 and 660 nm.

An improved and more stable CPC reagent system is formulated by using a bicarbonate or carbonate buffer. The bicarbonate or carbonate buffer system is selected from sodium carbonate, sodium bicarbonate, potassium carbonate, potassium bicarbonate, lithium carbonate and lithium bicarbonate.

The calcium reagent is composed of the following two components. A calcium dye reagent containing o-cresolphthalein complexone and 8-quinolol sulfate to reduce magnesium interference and the above carbonate or bicarbonate buffer at a pH of 9.0 to 10.5 containing nonylphenoxypoly(ethyleneoxy) ethanol and a salt of a cholic acid to minimize lipemia interference. A calcium working reagent is prepared by combining these two components in any of a number of ratios, preferably equal volumes of the calcium dye reagent and buffer are used.

Lipemic interference is minimized by using a surfactant as described below and salt of a cholic acid. Preferably, Igepal® CO-530 and sodium cholate can be used to diminish the turbidity of lipemic specimens and to enhance metal-dye complex formation. The surfactant is nonionic and non-electrolytic composition which is not subject to hydrolysis by aqueous solutions of acid or alkali. The preferred surfactant is a nonylphenoxypoly(ethyleneoxy)ethanol represented by the structural formula, 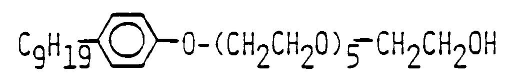
This particular surfactant is commercially available as Igepal® CO-530 surfactant from the GAF Corporation, Wayne, New Jersey. The surfactant is a clear viscous liquid which can exhibit slight solidification on cooling however, upon warming with agitation can be restored to an original condition.

The salt of cholic acid is necessary for the clarification of lipid turbidity. It is been found that the addition of a salt of cholic acid is required to solubilize the surfactant in water. Therefore, the method for clarifying lipid turbidity in a calcium reaction mixture for clinical assay requires the presence of both the surfactant and a salt of cholic acid.

The salt of cholic acid is preferably sodium cholate; however, other salts of cholic acid can be employed. The amount of the salt of cholic acid which must be employed with the surfactant has not been found to be particularly critical. Instead, the mere simultaneous presence of both compositions in the reaction mixture have been found to be effective for clarifying lipid turbidity.

In yet another preferred embodiment of the present invention, sodium azide can be employed as a preservative.

A typical reagent composition according to the present invention consists of a dye reagent comprising 0.09 g/l o-cresolphthalein complexone and 2.16 g/l 8-quinolinol sulfate and a buffer reagent comprising 0.2 mole/l of sodium carbonate,6 ml/l of Igepal® CO-530 and 4g/l sodium cholate.

The pH of the buffer is adjusted to between 9.0 and 10.5. Preferably, the pH is adjusted to 10.

The procedure for performing the spectrophotometric determination of calcium in plasma or serum with the improved CPC reagent comprises mixing equal volumes of both the dye and carbonate buffer, treating a sample with this mixture and monitoring a change in absorbance of the spectra of the mixture.

### EXAMPLE 1

A liquid calcium reagent according to the present invention was prepared having the following ingredients:

| | |
|---|---|
| Buffer: | sodium carbonate 0.2 mole/l |
| | Igepal® CO-530 6.0 ml/l |
| | sodium cholate 4.0 g/l (adjusted to a pH of 9.8) |
| Dye: | o-cresolphthalein complexion 0.09 g/l |
| | 8-quinolinol sulfate 2.16 g/l |

The buffer and dye components were mixed in equal volumes and used as the calcium reagent in the determination of calcium in serum samples on an Abbott Spectrum analyzer.

### EXAMPLE 2

The calcium reagent prepared in Example 1 was compared to a calcium reagent having a CAPS (3-cyclohexylamino)-1-propanesulfonic acid) buffer system instead of the carbonate buffer system for stability. Below is a comparison of the stabilities of these two reagents.

| WORKING REAGENT STABILITY | | |
|---|---|---|
| Temperature | CAPS Buffer Calcium Reagent | Carbonate Buffer Calcium Reagent |
| 18 to 28° C | 3 days | 10 days |
| 4 to 8° C | not determined | 1 month |

Working reagents using both buffer systems were prepared and stored at the indicated temperatures. Reagent performance was determined at several intervals. When reagent performance was deemed unacceptable (linearity, accuracy, etc.) the reagents were re-calibrated and retested. The times indicated refer to the longest time period during which the regent was functional (failure indicates an ability to calibrate the reagent).

The above reagents were also compared with respect to calibration interval. Both reagents were calibrated when first prepared. The reagents were tested on a regular basis until the reagent performance failed established specifications thereby requiring recalibration. This length of time between first calibration requirement is the calibration interval and is given below:

| CALIBRATION INTERVAL | | |
|---|---|---|
| Storage Temperature | CAPS Buffer Reagent | Carbonate Buffer Reagent |
| 18-28° C | 8 hours | 5 days |

## Claims

1. A stable calcium reagent for the spectrophotometric determination of calcium in a test sample, said stable calcium reagent characterized by:
(a) a dye component including:
(i) o-cresolphthalein complexone; and
(ii) a magnesium-interference reducing amount of 8-quinolinol sulfate; in admixture with
(b) a buffer component consisting essentially of:
(i) a reagent-stability improving amount of a carbonate or bicarbonate buffer selected from the group consisting of sodium carbonate, sodium bicarbonate, potassium carbonate, potassium bicarbonate, lithium carbonate and lithium bicarbonate; and
(ii) a lipid-turbidity reducing amount of nonylphenoxypoly(ethyleneoxy) ethanol in combination with a salt of a cholic acid, said buffer component having a pH of from 9.0 to 10.5.

2. The calcium reagent of Claim 1 wherein said dye component (a) and said buffer component (b) comprise approximately equal volumes in said stable calcium reagent.

3. The calcium reagent of Claim 1 wherein said dye component (a) comprises 0.09 grams per liter of (i) and 2.16 grams per liter of (ii).

4. The calcium reagent of Claim 3 wherein said buffer component (b) comprises 0.2 moles per liter of (i) sodium carbonate and as (ii) 6 milliliters per liter of nonylphenoxypoly(ethyleneoxy) ethanol and 4 grams per liter of sodium cholate.

5. The calcium reagent of Claim 1 wherein the salt of cholic acid is sodium cholate.

6. A method for improving the stability of a calcium dye reagent, which comprises o-cresolphthalein complexone and a magnesium-interference reducing amount of 8-quinolinol sulfate, for the spectrophotometric determination of calcium in a test sample, said method characterized by admixing a buffer component with said reagent, said buffer component consisting essentially of (i) a reagent-stability improving amount of a carbonate or bicarbonate buffer selected from the group consisting of sodium carbonate, sodium bicarbonate, potassium carbonate, potassium bicarbonate, lithium carbonate and lithium bicarbonate and (ii) a lipid-turbidity reducing amount of nonylphenoxypoly(ethyleneoxy) ethanol in combination with a salt of a cholic acid, said buffer component having a pH of from 9.0 to 10.5.

7. The method of Claim 6 wherein said calcium reagent and said buffer component are admixed in approximately equal volumes.

8. The method of Claim 6 wherein said calcium reagent comprises 0.09 grams per liter of o-cresolphthalein complexone and 2.16 grams per liter of 8-quinolinol sulfate.

9. The method of Claim 8 wherein said buffer component comprises 0.2 moles per liter of sodium carbonate and 6 milliliters per liter of nonylphenoxypoly(ethyleneoxy) ethanol and 4 grams per liter of sodium cholate.

10. The method of Claim 6 wherein the salt of cholic acid is sodium cholate.

## Patentansprüche

1. Ein stabiles Kalziumreagens zur spektrophotometrischen Bestimmung von Kalzium in einer Testprobe, wobei das stabile Kalziumreagens gekennzeichnet ist durch:
(a) eine Farbstoffkomponente bestehend aus:
(i) o-Kresolphthalein-Komplexbildner und
(ii) einem die Magnesiuminterferenz-vermindernden Anteil 8-Chinolinolsulfat in Mischung mit
(b) einer Pufferkomponente bestehend im wesentlichen aus:
(i) einem die Reagensstabilität-verbessernden Anteil an Carbonat- oder Bicarbonatpuffer, gewählt aus der Gruppe, bestehend aus Natriumcarbonat, Natriumbicarbonat, Kaliumcarbonat, Kaliumbicarbonat, Lithiumcarbonat und Lithiumbicarbonat und
(ii) einem die Lipidtrübungs-vermindernden Anteil an Nonylphenoxypoly(ethylenoxy) ethanol in Verbindung mit dem Salz einer Cholsäure, wobei die Pufferkomponente einen pH-Wert von 9,0 bis 10,5 hat.

2. Das Kalziumreagens nach Anspruch 1, wobei die Farbstoffkomponente (a) und die Pufferkomponente (b) ungefähr gleiche Volumina im stabilen Kalziumreagens haben.

3. Das Kalziumreagens nach Anspruch 1, wobei die Farbstoffkomponente (a) 0,09 g/l (i) und 2,16 g/l (ii) enthält.

4. Das Kalziumreagens nach Anspruch 3, wobei die Pufferkomponente (b) 0,2 Mol/l (i) Natriumcarbonat, und als (ii) 6 ml/l Nonylphenoxpoly(ethylenoxy)ethanol und 4 g/l Natriumcholat enthält.

5. Das Kalziumreagens nach Anspruch 1, wobei das Salz der Cholsäure Natriumcholat ist.

6. Ein Verfahren zur Verbesserung der Stabilität eines Kalziumfarbstoffreagens, das einen o-Kresolphthalein-Komplexbildner und einen die Magnesiuminterferenz-vermindernden Anteil an 8-Chinolinolsulfat enthält, zur spektrophotometrischen Messung von Kalzium in einer Probe, wobei das Verfahren gekennzeichnet ist durch Hinzumischen einer Pufferkomponente zum Reagens, die Pufferkomponente bestehend im wesentlichen aus (i) einem Reagensstabilitäts-verbessernden Anteil eines Carbonat- oder Bicarbonatpuffers, gewählt aus der Gruppe, bestehend aus Natriumcarbonat, Natriumbicarbonat, Kaliumcarbonat, Kaliumbicarbonat, Lithiumcarbonat und Lithiumbicarbonat und (ii) einem die Lipidtrübungsvermindernden Anteil an Nonylphenoxypoly(ethylenoxy)ethanol in Verbindung mit dem Salz einer Cholsäure, wobei der Puffer einen pH-Wert von 9,0 bis 10,5 hat.

7. Das Verfahren nach Anspruch 6, wobei das Kalziumreagens und die Pufferkomponente in ungefähr gleichen Volumina gemischt werden.

8. Das Verfahren nach Anspruch 6, wobei das Kalziumreagens 0,09 g/l eines o-Kresolphthalein-Komplexbildners und 2,16 g/l 8-Chinolinolsulfat enthält.

9. Verfahren nach Anspruch 8, wobei die Pufferkomponente 0,2 Mol/l Natriumcarbonat und 6 ml/l Nonylphenoxypoly(ethylenoxy)ethanol und 4 g/l Natriumcholat enthält.

10. Verfahren nach Anspruch 6, wobei das Salz der Cholsäure Natriumcholat ist.

## Revendications

1. Réactif du calcium stable pour le dosage spectrophotométrique du calcium dans un échantillon à tester, ledit réactif du calcium stable état caractérisé par :
(a) un composant colorant incluant :
(i) la complexone o-crésolphtaléine ; et
(ii) une quantité de sulfate de 8-quinoléinol réduisant l'interférence due au magnésium ; en mélange avec
(b) un composant tampon comprenant essentiellement :
(i) une quantité, améliorant la stabilité du réactif, de tampon carbonate ou bicarbonate, sélectionné dans le coupe comprenant le carbonate de sodium, le bicarbonate de sodium, le carbonate de potassium, le bicarbonate de potassium, le carbonate de lithium et le bicarbonate de lithium ; et
(ii) une quantité de nonylphénoxypoly(éthylèneoxy) éthanol réduisant l'opacité due aux lipides en association avec un sel d'acide cholique, ledit composant tampon ayant un pH allant de 9,0 à 10,5.

2. Réactif du calcium de la revendication 1, dans lequel ledit composant colorant (a) et ledit composant tampon (b) participent approximativement en volumes égaux audit réactif du calcium stable.

3. Réactif du calcium de la revendication 1, dans lequel ledit composant colorant (a) comprend 0,09 gramme par litre de (i) et 2,16 grammes par litre de (ii).

4. Réactif du calcium de la revendication 3, dans lequel ledit composant tampon (b) comprend 0,2 mole par litre de carbonate de sodium en tant que (i) et 6 millilitres par litre de nonylphénoxypoly(éthylèneoxy)éthanol et 4 grammes par litre de cholate de sodium en tant que (ii).

5. Réactif du calcium de la revendication 1, dans lequel le sel d'acide cholique est du cholate de sodium.

6. Méthode pour améliorer la stabilité d'un réactif coloré du calcium, qui comprend la complexone o-crésolphtaléine et une quantité de sulfate de 8-quinoléinol réduisant l'interférence due au magnésium pour le dosage spectrophotométrique du calcium dans un échantillon à tester, ladite méthode étant caractérisée par le mélange d'un composant tampon audit réactif, ledit composant tampon comprenant essentiellement (i) une quantité, améliorant la stabilité du réactif de tampon carbonate ou bicarbonate sélectionné dans le groupe comprenant le carbonate de sodium, le bicarbonate de sodium, le carbonate de potassium, le bicarbonate de potassium, le carbonate de lithium et le bicarbonate de lithium et (ii) une quantité de nonylphénoxypoly(éthylèneoxy) éthanol réduisant l'opacité due aux lipides en association avec un sel d'acide cholique, ledit composant tampon ayant un pH allant de 9,0 à 10,5.

7. Méthode de la revendication 6, dans laquelle ledit réactif du calcium et ledit composant tampon sont mélangés en volumes approximativement égaux.

8. Méthode de la revendication 6, dans laquelle ledit réactif du calcium comprend 0,09 gramme par litre de complexone o-crésolphtaléine et 2,16 grammes par litre de sulfate de 8-quinoléinol.

9. Méthode de la revendication 8, dans laquelle ledit composant tampon comprend 0,2 mole par litre de carbonate de sodium et 6 millilitres par litre de nonylphénoxypoly(éthylèneoxy)éthanol et 4 grammes par litre de cholate de sodium.

10. Méthode de la revendication 6, dans laquelle le sel d'acide cholique est du cholate de sodium.
